# EUROPÄISCHE PATENTANMELDUNG

(11) **EP 0 548 895 A2**
(43) Veröffentlichungstag der Anmeldung: **30.06.1993**
(21) Anmeldenummer: 92121743.6
(22) Anmeldetag: 21.12.1992
(51) Int. Cl.: C07D 487/14, C07D 475/04

(54) **Verfahren zur Herstellung von diastereoisomerenreinen Tetrahydrofolaten**

(30) Priorität: 21.12.1991 CH 3799/91
(71) Anmelder: SAPEC S.A. FINE CHEMICALS, CH-6903 Lugano (CH)
(72) Erfinder: Jéquier,Pascal, CH-6815 Melide (CH); Marazza,Fabrizio, CH-6924 Sorengo (CH)
(74) Vertreter: Zink-Wild, Markus Peter

(57) **Zusammenfassung**

Das erfindungsgemässe Verfahren zur Herstellung von (6R)-N(5),N(10)-Methenyl-5,6,7,8-tetrahydrofolsäure-salzen der Formel I und der entsprechenden Säureadditionssalze, und der entsprechenden inneren Salze ist dadurch gekennzeichnet, dass man ein in Wasser oder ein in einer wässrigen Pufferlösung gelöstes Ammonium- oder Alkalimetallsalz von (6RS)-N(10)-Formyl-5,6,7,8-tetrahydrofolsäure der Formel II, vorzugsweise unter einer Inertgasatmosphäre, so lange mit wenigstens einer Säure S₁ vermischt, bis ein pH-Wert im Bereich von 5,5 bis 7,5 erhalten wird, dann zum so erhaltenen Gemisch ein wasserlösliches Erdalkalimetallsalz hinzugibt, anschliessend den entstandenen Festkörper A isoliert, diesen Festkörper dann in Wasser suspendiert und solange Säure S₂ hinzugibt, bis sich der gemessene pH-Wert des entstandenen Gemisches auf einen Wert im Bereich von 2,0 bis 1,0, vorzugsweise 1,5, stabilisiert hat, und den erhaltenen Festkörper der Formel I abtrennt.

## Beschreibung

Die vorliegende Erfindung betrifft ein Verfahren zur Herstellung von diastereoisomerenreinen Tetrahydrofolaten.

Verfahren zur Herstellung von reduzierten Folaten mit einheitlicher (6S)- oder (6R)-Konfiguration sind in der internationalen Patentanmeldung PCT/FR 91/00185 sowie in der Europäischen Patentanmeldung Nr. 91 105 715.6, Veröffentlichungs-Nr. 0 455 013 A1, beschrieben. Hierin ist auch der weitere Stand der Technik beschrieben. Diese Angaben werden mit diesem Hinweis als auch hierin offenbart betrachtet.

Es ist ein Ziel der vorliegenden Erfindung, ein einfaches, billiges, im industriellen Massstab durchführbares und umweltfreundliches Verfahren zur Herstellung von diastereoisomerenreinen Tetrahydrofolaten zur Verfügung zu stellen.

Das erfindungsgemässe Verfahren zur Herstellung von (6R)-N(5),N(10)-Methenyl-5,6,7,8-tetrahydrofolsäure-salzen der Formel I 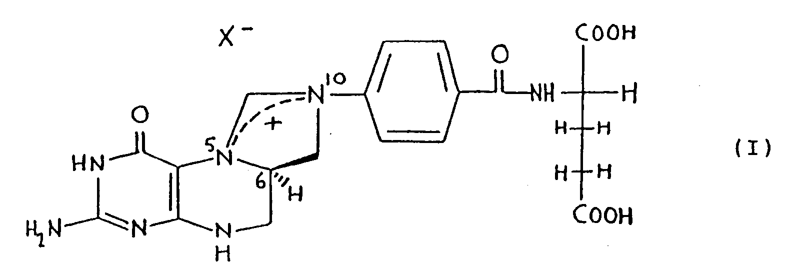
worin X ein Anion oder ein Aequivalent eines Anions bedeutet, und der entsprechenden Säureadditionssalze, und der entsprechenden inneren Salze,
ist dadurch gekennzeichnet, dass man ein in Wasser oder ein in einer wässrigen Pufferlösung gelostes Ammonium- oder Alkalimetallsalz von (6RS)-N(10)-Formyl-5,6,7,8-tetrahydrofolsäure der Formel II 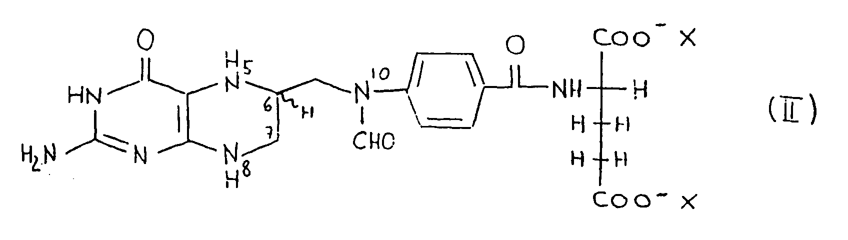
worin X ein Alkalimetallkation oder NH₄⁺ bedeutet,
vorzugsweise unter einer Inertgasatmosphäre, so lange mit wenigstens einer Säure S₁ vermischt, bis ein pH-Wert im Bereich von 5,5 bis 7,5 erhalten wird,
dann zum so erhaltenen Gemisch ein wasserlösliches Erdalkalimetallsalz hinzugibt,
anschliessend den entstandenen Festkörper A isoliert,
diesen Festkörper dann in Wasser suspendiert und solange Säure S₂ hinzugibt, bis sich der gemessene pH-Wert des entstandenen Gemisches auf einen Wert im Bereich von 2,0 bis 1,0, vorzugsweise 1,5, stabilisiert hat, und
den erhaltenen Festkörper der Formel I abtrennt.

Bevorzugte Ausführungsformen dieses Verfahrens sind in den abhängigen Ansprüchen definiert.

In der vorliegenden Erfindung wird 5,6,7,8-Tetrahydrofolsäure manchmal mit THF abgekürzt.

Es wurde insbesondere gefunden, dass das Ansäuern einer wässrigen Lösung eines Ammonium- oder eines Alkalimetallsalzes von (6RS)-N(10)-Formyl-THF auf einen pH-Wert im bevorzugten Bereich von 6,7 bis 7,0 - beispielsweise mittels kontinuierlicher Zugabe von Säure bis zu einem konstanten pH-Wert -, gefolgt von der nachfolgenden Hinzugabe eines wasserlöslichen Erdalkalimetallsalzes, die Fällung eines Festkörpers A bewirkt.

Das Ausgangsmaterial (6RS)-N(10)-Formyl-THF kann beispielsweise aus (6RS)-Methenyl-THF.Cl⁻ gemäss der Schweizerischen Patentanmeldung Nr. 02 933/91-9 und hierin offenbarten Literaturstellen hergestellt werden.

Wird der oben genannte Festkörper A in einem geeigneten Lösungsmittel gelöst, beispielsweise in Wasser oder Formamid, und mittels HPLC analysiert, so zeigt der erhaltene Peak eine Retensionszeit, welche mit derjenigen identisch ist, die erhalten wird mit Methenyl-THF. Deswegen kann man annehmen, dass der Festkörper A die Teilstruktur von Methenyl-THF aufweist.

Zudem weisen die Massenspektren (FAB/Matrix = Thioglyzerin) den gleichen m⁺ = 456 auf.

Die Elementaranalyse des Festkörpers A zeigt die Anwesenheit von einem halben Equivalent Erdalkalimetallkation (Ca²⁺ oder Mg²⁺) und ist in Uebereinstimmung mit der folgenden Summenformel:

C₂₀H₂₀N₇O₆Me_{0,5}

wobei Me ein Erdalkalimetallkation (z.B. Ca²⁺ oder Mg²⁺) bedeutet.

Im Folgenden wird auf die Unterschiede der ¹H-NMR-Spektren (400 MHz/aufgenommen in d₃-Formamid) von (6R)-Methenyl-THF.Cl⁻ und dem Festkörper A (Ca²⁺ und Mg²⁺ Salz) hingewiesen.

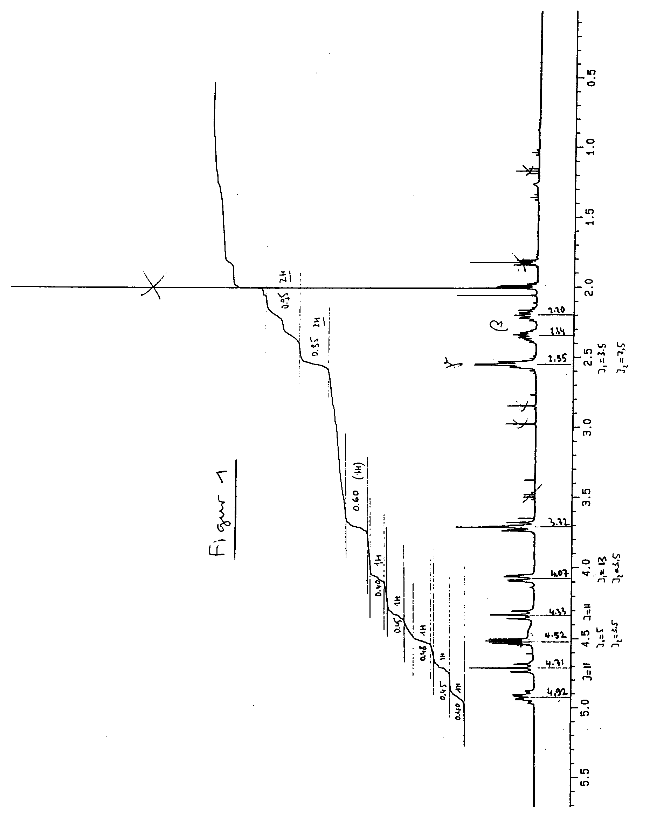

Figur 1 zeigt einen Teil des ¹H-NMR-Spektrums des Ca²⁺-Salzes des Festkörpers A.

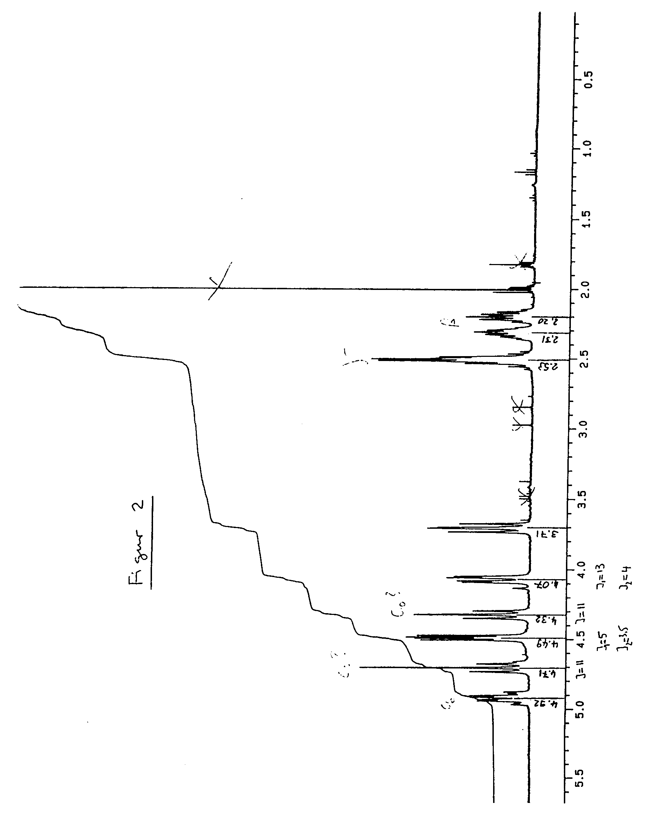

Figur 2 zeigt einen Teil des ¹H-NMR-Spektrums des Mg²⁺-Salzes des Festkörpers A.

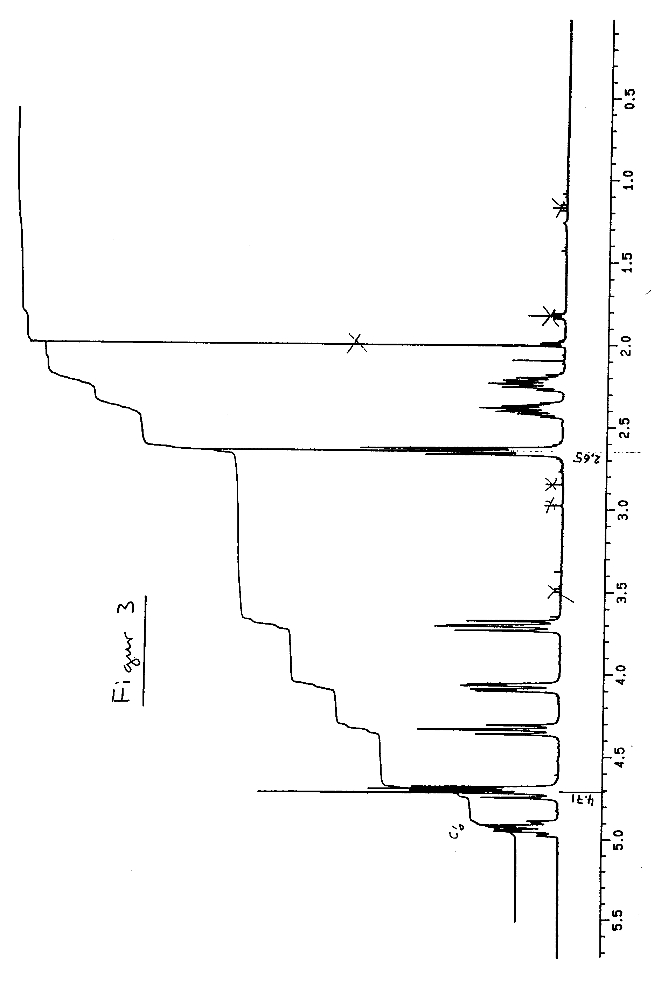

Figur 3 zeigt einen Teil des ¹H-NMR-Spektrums von (6R)-Methenyl-THF.Cl⁻.

In den Spektren der genannten Ca²⁺- und Mg²⁺-Salze geben alle 15 an C-Atome gebundene Wasserstoffatome scharfe, getrennte Signale.

Im Spektrum von (6R)-Methenyl-THF.Cl⁻ beobachtet man ein Multiplet bei 4.71 ppM, welches integriert 2 Protonen entspricht.

Bei den genannten Ca²⁺- und Mg²⁺-Salzen beobachtet man hingegen je zwei getrennte Signale:
4,52 (1H) und 4,71 (1H) für das Ca²⁺-Salz respektive 4,49 (1H) und 4,71 (1H) für das Mg²⁺-Salz.

Ausserdem ergibt die γ-CH₂ Gruppe der Glutamatseitenkette im Falle von (6R)-Methenyl-THF.Cl⁻ einen scharfen Triplet bei 2.65 ppM. Im Falle der Ca²⁺- und Mg²⁺-Salze beobachtet man dagegen für diese Gruppe je ein Multiplet bei 2.55 ppM, resp. 2.53 ppM.

Obige Daten lassen darauf schliessen, dass der Festkörper A die Struktur der Formel IV aufweist.

Somit sind völlig überraschend hemi-Erdalkalimetallsalze von N(5),N(10)-Methenyl-THF gefunden worden.

Diese Erdalkalimetallsalze zeigen überraschenderweise ein selektives Kristallisationsverhalten, welches verwendet werden kann zur Anreicherung der (6R)-Form.

Wird ein solches Salz zur N(5)-Methyl-THF reduziert und anschliessend auf einer chiralen Säule analysiert, so wird eine diastereoisomere Reinheit der (6S)-Form von 85 bis 95 % festgestellt.

Die Säure S₂ muss eine starke Säure sein, und sie darf mit einem Erdalkalimetallkation keinen Niederschlag geben. Als Säure S₂ sind Schwefelsäure und Phosphorsäure ungeeignet. Bevorzugt ist Salzsäure, insbesondere wässrige Salzsäure.

Behandelt man die genannten neuen Erdalkalimetallsalze mit wässriger Salzsäure (pH etwa 1), so erhält man die bekannte Verbindung (6R)-N(5),N(10)-Methenyl-THF.Cl⁻ der Formel I; vergleiche die Schweizerische Patentanmeldung Nr. 02 794/91-0.

Die Verbindung der Formel I kann gemäss bekannten Verfahren entweder mittels Reduktion in (6S)-N(5)-Methyl-THF.Ca²⁺ (siehe: White, Bailey and Goldman, J. Biol. Chem., (1978), 253,242) oder mittels Hydrolyse in (6S)-N(5)-Formyl-THF.Ca²⁺ umgewandelt werden (siehe: CH PS 305 574).

Es ist auch gefunden worden, dass die neuen Erdalkalimetallsalze direkt in (6S)-Erdalkalimetall-Folinate umgewandelt werden können, insbesondere in (6S)-Calcium-Folinat.

Die nachfolgenden Beispiele dienen der Illustration der vorliegenden Erfindung.

### Beispiel 1

Zu einer Suspension von 50 g (6RS)-Methenyl-THF.Cl⁻ in 450 ml Wasser, gerührt unter einer Stickstoffatmosphäre bei einer Temperatur von 50°C, wurde tropfenweise 20%-ige NaOH solange zugegeben, bis ein stabiler gemessener pH-Wert von 8.3 erreicht war.

Eine HPLC-Analyse des erhaltenen Gemisches zeigte kein Methenyl-THF mehr, dafür aber die Anwesenheit eines neuen Peaks, welcher dem gebildeten N(10)-Formyl-THF.Na₂ zugeschrieben wurde.

Zur erhaltenen Lösung wurde dann bei einer Temperatur von 40°C solange 30%-ige, wässrige Essigsäure zugegeben, bis das Gemisch einen pH-Wert von 6.8 aufwies. Dann wurden 12 g Calciumacetat zugegeben, und das Gemisch wurde weiter gerührt bei der gleichen Temperatur, wobei ein Produkt auszukristallisieren begann. Danach wurden während 3 Stunden 8 ml 30%-ige, wässrige Essigsäure zugetropft. Das Gemisch zeigte danach einen pH-Wert von 6.8.

Das so erhaltene Kristallisat wurde abfiltriert, 2 mal mit Wasser und zweimal mit Aceton gewaschen, und schliesslich bei einer Temperatur von 60°C unter vermindertem Druck getrocknet, wobei 12 g eines gelben Feststoffes isoliert wurden (25 % w/w Ausbeute).

Die HPLC Analyse an einer reversed phase (RP)-Säule Zeigte eine 95%-ige chemische Reinheit.

**IR (KBr):** 3392, 3220, 3095, 2930, 1650, 1601, 1559, 1510, 1450, 1400, 1341, 1318, 1289, 1243, 757 cm⁻¹.
- **MS (FAB/Matrix: Thioglyzerin)**:: m⁺ = 456
- **UV (20 mg/l in H₂O/HCONH₂ 99:1):**: λₘₐₓ=360.5 nm
λₘᵢₙ=303.5 nm
Aₘₐₓ/Aₘᵢₙ = 4.24

| Elementaranalyse | | | | |
|---|---|---|---|---|
| | %C | %H | %N | %Ca |
| Theorie für C₂₀H₂₀N₇O₆Ca_{0.5}x4 H₂O | 43.97 | 5.17 | 17.95 | 3.67 |
| Experimentelle Werte | 43.94 | 4.85 | 17.65 | 3.21 |

Zur Feststellung der diastereoisomeren Reinheit wurden 15 mg der oben genannten Kristalle in 1,5 ml einer 1,8 %-igen Lösung von p-Toluolsulfonsäure gelöst. Danach wurden 6 mg NaBH₃CN zugegeben und das Gemisch wurde während 30 Minuten bei Raumtemperatur gerührt. Dabei wurden die genannten Kristalle vollständig in N(5)-Methyl-THF umgewandelt. Die Analyse der letzteren Verbindung mittels einer chiralen HPLC-Säule (RESOLVOSIL) zeigte eine diastereoisomere Reinheit von 90 % (6S).

¹H-NMR Spektrum (400 MHz/in d₃-Formamid / Ref.: TMS):
9.66 (1H,s); 8.06 (2H,d); 7.48 (2H,d); 4.92 (1H,dq); 4.71 (1H,t); 4.51 (1H,dd); 4.33 (1H,t); 4.07 (1H,dd); 3.71 (1H,m); 2.55 (2H,dt); 2.34 (1H,m); 2.20 (1H,m).

### Beispiel 2

Ausgehend von 50 g (6RS)-Methenyl-THF.Cl⁻, in analoger Weise wie im Beispiel 1 beschrieben, wurde eine Lösung von N(10)-Formyl-THF.Na₂ hergestellt. Diese wurde bei Raumtemperatur mittels tropfenweiser Zugabe von 30 %-iger, wässriger Essigsäure auf einem pH-Wert von 6.9 gebracht. Danach wurden 15 g festes Magnesiumacetat-tetrahydrat zugegeben, und das Gemisch wurde bei der gleichen Temperatur gerührt, wobei sich ein kristalliner Festkörper auszuscheiden begann. Gleichzeitig hielt man den pH-Wert zwischen 6.8 und 7.0 mittels tropfenweiser Zugabe von 8 ml 30 %-iger, wässriger Essigsäure während 10 Stunden.

Die erhaltene Suspension wurde dann bis auf eine Temperatur von 0°C abgekühlt und während einer Stunde bei dieser Temperatur gerührt. Das Kristallisat wurde mittels Filtration isoliert, 3 mal mit H₂O und 2 mal mit Aceton gewaschen und während 2 Stunden unter vermindertem Druck bei einer Temperatur von 60°C getrocknet.

Ausbeute: 14 g (28 % w/w) eines gelben Festkörpers.

Die HPLC Analyse an einer RP-Säule zeigte eine 95%-ige chemische Reinheit.
**IR (KBr):** 3340, 3110, 2950, 1630, 1600, 1560, 1508, 1450, 1405, 1318, 1289, 1243, 763, 657, 612 cm⁻¹.
- **MS (FAB/Matrix: Thioglyzerin):**: m⁺ = 456
- **UV (20 mg/l in H₂O/HCONH₂ 99:1):**: λₘₐₓ=359 nm
λₘᵢₙ=301 nm
Aₘₐₓ/Aₘᵢₙ = 4.24
- **Komplexometrische Titration von Magnesium:**: 2.35 % Mg
(theoretischer Wert korrigiert für den H₂O Gehalt: 2.31 %).

Zur Feststellung der diastereoisomeren Reinheit wurden 15 mg der oben genannten Kristalle in 1,5 ml einer 1,8 %-igen Lösung von p-Toluolsulfonsäure gelöst. Danach wurden 6 mg NaBH₃CN zugegeben und das Gemisch wurde während 30 Minuten bei Raumtemperatur gerührt. Dabei wurden die genannten Kristalle vollständig in N(5)-Methyl-THF umgewandelt. Die Analyse der letzteren Verbindung mittels einer chiralen HPLC-Säule (RESOLVOSIL) zeigte eine diastereoisomere Reinheit von 88 % (6S).

¹H-NMR Spektrum (400 MHz/in d₃-Formamid / Ref.: TMS):
9.66 (1H,s); 8.06 (2H,d); 7.47 (2H,d); 4.92 (1H,dq); 4.71 (1H,t); 4.49 (1H,dd); 4.32 (1H,t); 4.07 (1H,dd); 3.71 (1H,m); 2.53 (2H,m); 2,31 (1H,M); 2.20 (1H,m).

### Beispiel 3

20 g des gemäss Beispiel 1 hergestellten Produktes wurden in 200 ml Wasser bei einer Temperatur von 30°C suspendiert. Danach wurde solange 18%-ige wässrige Salzsäure hinzugetropft, bis sich der gemessene pH-Wert des entstandenen Gemisches auf einen Wert von 1,3 stabilisiert hat.

Dieses Gemisch wurde bei einer Temperatur von 30°C während 30 Minuten gerührt.

Der Festkörper wurde abfiltriert, 2 mal mit Wasser und 2 mal mit Aceton gewaschen, und schliesslich bei einer Temperatur von 60°C unter vermindertem Druck getrocknet.

Man erhielt 18,4 g eines gelben Festkörpers, nämlich (6R)-N(5),N(10)-Methenyl-THF-chlorid.

Die HPLC Analyse an einer RP-Säule zeigte eine 100%-ige chemische Reinheit (Detektion bei 354 nm).

Es wurde die gleiche diastereoisomere Reinheit, wie in Beispiel 1 beschrieben, erhalten.

### Beispiel 4

### Umwandlung (6R)-Hemicalcium-Salz in (6S)-Calciumfolinat

100.0 g des Hemicalcium-Salzes von (6R)-N(5),N(10)-Methenyl-THF, erhalten wie in Beispiel 1 beschrieben, wurden in 800 ml Wasser unter einer Stickstoffatmosphäre bei einer Temperatur von 80°C erwärmt.

Der pH-Wert des Gemisches wurde während der Reaktion mittels kontinuierlicher Zugabe einer 25%-igen wässrigen Suspension von Calciumhydroxid bei 5,8 gehalten.

Nach 12-stündigem Erwärmen bei 80° C wurde die erhaltene (6S)-N(5)-Formyl-THF Lösung auf eine Temperatur von 30°C abgekühlt, mit 5,0 g Aktivkohle behandelt und mittels Zugabe einer wässrigen Calciumhydroxid Suspension auf einen pH-Wert von 7,5 gebracht.

Das erhaltene Gemisch wurde dann über eine Schicht Celite filtriert und unter vermindertem Druck und bei einer Temperatur von 50°C auf ein Volumen von 600 ml eingeengt.

Die erhaltene Lösung wurde dann innerhalb von 32 Stunden langsam und kontrolliert von 50°C auf Raumtemperatur abgekühlt, wobei das gewünschte (6S)-Calcium-Folinat auskristallisierte.

Das Produkt wurde abfiltriert und aus Wasser nochmals kristallisiert.

Das mittels Filtration erhaltene Kristallisat wurde einmal mit Wasser und einmal mit 94%-igem Ethanol gewaschen und unter vermindertem Druck bei einer Temperatur von 50°C getrocknet.

Auf diese Weise wurden 42,0 g des reinen (6S)-Calcium-Folinats isoliert.

Die HPLC Analyse an einer RP-Säule zeigte eine chemische Reinheit von 99,6 %.

Der Vergleich mit der Referenzsubstanz [USP Standard / (6RS)-Gemisch], ebenfalls mittels HPLC, ergab einen Gehalt von 97,3 %.

Die diastereoisomere Reinheit wurde auf einer chiralen HPLC Säule getestet (RESOLVOSIL), wobei ein Wert von 99,3 % ermittelt wurde.

Bestimmung des Wassergehaltes nach Karl Fischer: 14,0 %.

Gaschromatographische Bestimmung von Ethanol: 0,6 %.

Komplexometrische Bestimmung von Calcium: 8,0 % (Theorie: 7,84 %).

## Patentansprüche

1. Verfahren zur Herstellung von (6R)-N(5),N(10)-Methenyl-5,6,7,8-tetrahydrofolsäure-salzen der Formel I 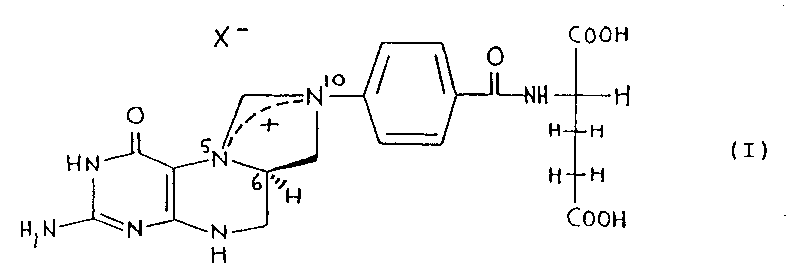 worin X ein Anion oder ein Aequivalent eines Anions bedeutet,
und der entsprechenden Säureadditionssalze, und der entsprechenden inneren Salze,
dadurch gekennzeichnet, dass man ein in Wasser oder ein in einer wässrigen Pufferlösung gelöstes Ammonium- oder Alkalimetallsalz von (6RS)-N(10)-Formyl-5,6,7,8-tetrahydrofolsäure der Formel II 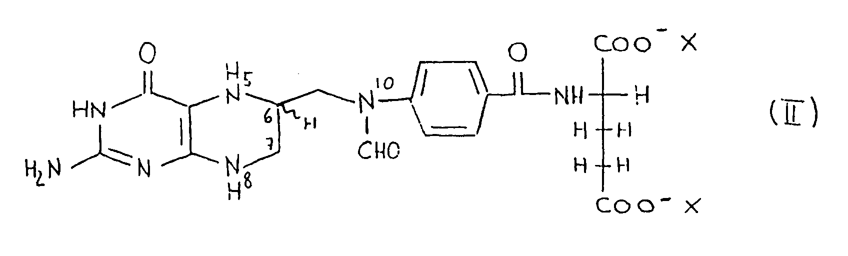 worin X ein Alkalimetallkation oder NH₄⁺ bedeutet,
vorzugsweise unter einer Inertgasatmosphäre, so lange mit wenigstens einer Säure S₁ vermischt, bis ein pH-Wert im Bereich von 5,5 bis 7,5 erhalten wird,
dann zum so erhaltenen Gemisch ein wasserlösliches Erdalkalimetallsalz hinzugibt,
anschliessend den entstandenen Festkörper A isoliert,
diesen Festkörper dann in Wasser suspendiert und solange Säure S₂ hinzugibt, bis sich der gemessene pH-Wert des entstandenen Gemisches auf einen Wert im Bereich von 2,0 bis 1,0, vorzugsweise 1,5, stabilisiert hat, und
den erhaltenen Festkörper der Formel I abtrennt.

2. Verfahren nach Anspruch 1, dadurch gekennzeichnet, dass das Anion X ein Chlorid oder ein Bromid ist.

3. Verfahren nach einem der Ansprüche 1 bis 2, dadurch gekennzeichnet, dass das Säureadditionssalz ein Hydrochlorid, ein Hydrobromid, ein organisches Sulfonat, beispielsweise Methansulfonat, ein Formiat, ein Oxalat, ein Maleinat, ein Trichloracetat, oder ein Trifluoracetat ist.

4. Verfahren nach einem der Ansprüche 1 bis 3, dadurch gekennzeichnet, dass die Säure S₁ ausgewählt ist aus der Gruppe, bestehend aus niedrigen, wasserlöslichen Carbonsäuren, insbesondere Ameisensäure und Essigsäure, und Halogenwasserstoffsäuren, insbesondere HCl und HBr, wobei die genannten Säuren vorzugsweise als wässrige Lösungen verwendet werden.

5. Verfahren nach einem der Ansprüche 1 bis 4, dadurch gekennzeichnet, dass die Hinzugabe der Säure S₁ und des Erdalkalimetallsalzes bei einer Temperatur im Bereich von 0°C bis 60°C, insbesondere von 10°C bis 40°C, erfolgt.

6. Verfahren nach einem der Ansprüche 1 bis 5, dadurch gekennzeichnet, dass das Erdalkalimetallsalz ein entsprechendes Salz einer niedrigen, wasserlöslichen Carbonsäure, insbesondere ein Formiat oder ein Acetat, oder ein Halogenid, insbesondere ein Chlorid und Bromid, ist, wobei die entsprechenden Calcium- und Magnesiumsalze bevorzugt sind.

7. Verfahren nach einem der Ansprüche 1 bis 6, dadurch gekennzeichnet, dass das Erdalkalimetallsalz in einer Menge von 0,5 bis 1,0, insbesondere 0,7, Molequivalenten, bezogen auf die verwendete Menge an Verbindung der Formel II, verwendet wird.

8. Verfahren nach einem der Ansprüche 1 bis 7, dadurch gekennzeichnet, dass man die Verbindungen der Formel II dadurch erhält, dass man zu einer wässrigen, gegebenenfalls einen Puffer enthaltenden Suspension eines (6RS)-Diastereoisomerengemisches eines N(5),N(10)-Methenyl-5,6,7,8-tetrahydrofolsäure-salzes der Formel III 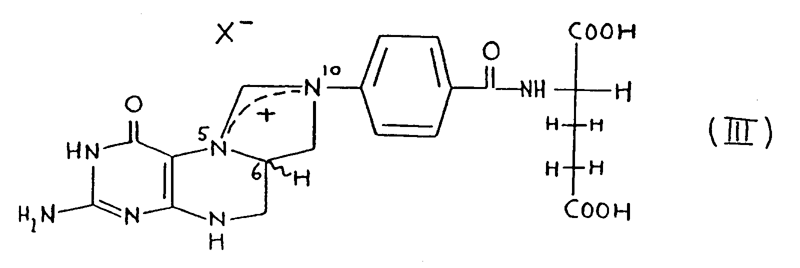 worin X weiter oben definiert ist,
oder eines entsprechenden Säureadditionssalzes oder des entsprechenden inneren Salzes
so lange eine entsprechende Base, vorzugsweise Natriumhydroxid oder Ammoniak, bei einer Temperatur im Bereich von 20°C bis 60°C, insbesondere bei 50°C, hinzugibt, bis ein stabiler pH-Wert im Bereich von 7,5 bis 9,0, insbesondere 8,3, erhalten wird.

9. Verfahren nach einem der Ansprüche 1 bis 8, dadurch gekennzeichnet, dass die Säure S₂ ausgewählt ist aus der Gruppe, bestehend aus Salzsäure, p-Toluolsulfonsäure, Methansulfonsäure, Trifluoressigsäure und Trichloressigsäure.

10. Verfahren zur Herstellung von (6R)-N(5),N(10)-Methenyl-5,6,7,8-tetrahydrofolsäure-hemi-Erdalkalimetallsalzen der Formel IV 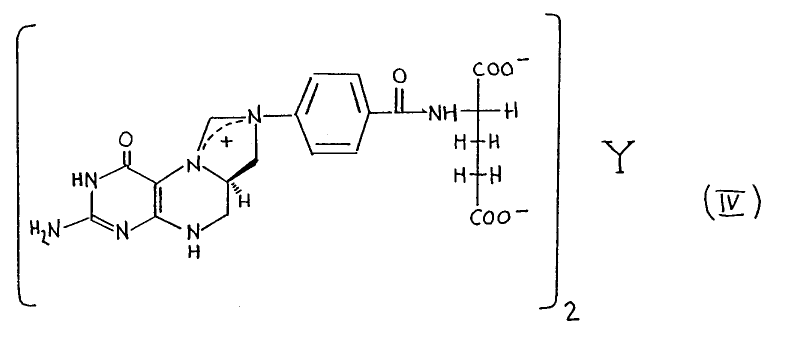 worin Y ein Erdalkalimetallkation bedeutet, und insbesondere Ca²⁺ oder Mg²⁺ ist,
dadurch gekennzeichnet, dass man ein in Wasser oder ein in einer wässrigen Pufferlösung gelöstes Ammonium- oder Alkalimetallsalz von (6RS)-N(10)-Formyl-5,6,7,8-tetrahydrofolsäure der Formel II 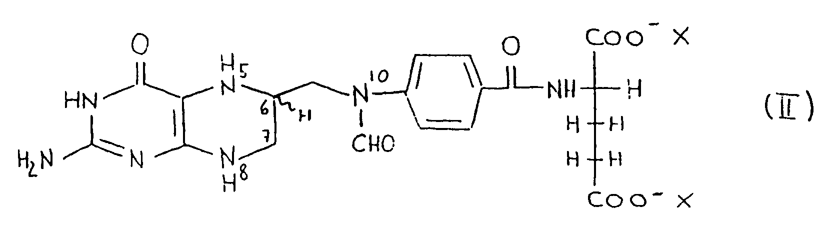 worin X ein Alkalimetallkation oder NH₄⁺ bedeutet,
vorzugsweise unter einer Inertgasatmosphäre, so lange mit wenigstens einer Säure S₁ vermischt, bis ein pH-Wert im Bereich von 5,5 bis 7,5 erhalten wird,
dann zum so erhaltenen Gemisch ein wasserlösliches Erdalkalimetallsalz hinzugibt, und
den entstandenen Festkörper der Formel IV isoliert.

11. Verfahren nach Anspruch 10, dadurch gekennzeichnet, dass das Anion X ein Chlorid oder ein Bromid ist.

12. Verfahren nach einem der Ansprüche 10 bis 11, dadurch gekennzeichnet, dass das Säureadditionssalz ein Hydrochlorid, ein Hydrobromid, ein organisches Sulfonat, beispielsweise Methansulfonat, ein Formiat, ein Oxalat, ein Maleinat, ein Trichloracetat, oder ein Trifluoracetat ist.

13. Verfahren nach einem der Ansprüche 10 bis 12, dadurch gekennzeichnet, dass die Säure S₁ ausgewählt ist aus der Gruppe, bestehend aus niedrigen, wasserlöslichen Carbonsäuren, insbesondere Ameisensäure und Essigsäure, und Halogenwasserstoffsäuren, insbesondere HCl und HBr, wobei die genannten Säuren vorzugsweise als wässrige Lösungen verwendet werden.

14. Verfahren nach einem der Ansprüche 10 bis 13, dadurch gekennzeichnet, dass die Hinzugabe der Säure S₁ und des Erdalkalimetallsalzes bei einer Temperatur im Bereich von 0°C bis 60°C, insbesondere von 10°C bis 40°C, erfolgt.

15. Verfahren nach einem der Ansprüche 10 bis 14, dadurch gekennzeichnet, dass das Erdalkalimetallsalz ein entsprechendes Salz einer niedrigen, wasserlöslichen Carbonsäure, insbesondere ein Formiat oder ein Acetat, oder ein Halogenid, insbesondere ein Chlorid und Bromid, ist, wobei die entsprechenden Calcium- und Magnesiumsalze bevorzugt sind.

16. Verfahren nach einem der Ansprüche 10 bis 15, dadurch gekennzeichnet, dass das Erdalkalimetallsalz in einer Menge von 0,5 bis 1,0, insbesondere 0,7, Molquivalenten, bezogen auf die verwendete Menge an Verbindung der Formel II, verwendet wird.

17. Verfahren nach einem der Ansprüche 10 bis 16, dadurch gekennzeichnet, dass man die Verbindungen der Formel II dadurch erhält, dass man zu einer wässrigen, gegebenenfalls einen Puffer enthaltenden Suspension eines (6RS)-Diastereoisomerengemisches eines N(5),N(10)-Methenyl-5,6,7,8-tetrahydrofolsäure-salzes der Formel III 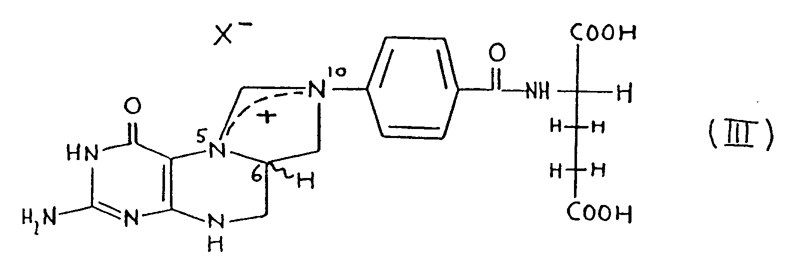 worin X weiter oben definiert ist,
oder eines entsprechenden Säureadditionssalzes oder des entsprechenden inneren Salzes
so lange eine entsprechende Base, vorzugsweise Natriumhydroxid oder Ammoniak, bei einer Temperatur im Bereich von 20°C bis 60°C, insbesondere bei 50°C, hinzugibt, bis ein stabiler pH-Wert im Bereich von 7,5 bis 9,0, insbesondere 8,3, erhalten wird.

18. N(5),N(10)-Methenyl-5,6,7,8-tetrahydrofolsäure-hemi-Erdalkalimetallsalze der Formel IV' 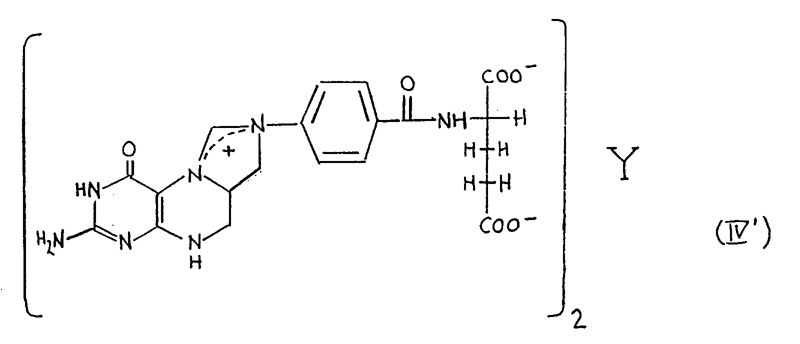 worin Y ein Erdalkalimetallkation bedeutet, und insbesondere Ca²⁺ oder Mg²⁺ ist,
in der Form des (6RS)-Diastereoisomerengemisches oder in der Form der einzelnen (6R)- oder (6S)-Diastereoisomeren.

19. Verwendung der Verbindungen der Formeln I als Ausgangsmaterialien zur Herstellung von (6S)-N(5)-Methyl-THF und (6S)-N(5)-Formyl-THF.

20. Verfahren zur Herstellung von (6S)-N(5)-Formyl-5,6,7,8-tetrahydrofolsäure-Erdalkalimetallsalzen der Formel V 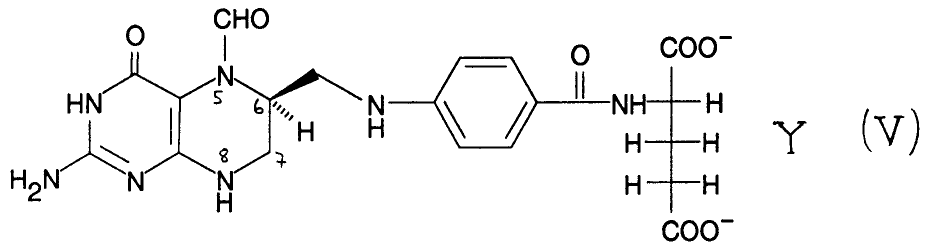 worin Y ein Erdalkalimetallkation bedeutet, und insbesondere Ca²⁺ oder Mg²⁺ ist,
dadurch gekennzeichnet, dass man ein in Wasser oder ein in einer wässrigen Pufferlösung gelöstes Ammonium- oder Alkalimetallsalz von (6RS)-N(10)-Formyl-5,6,7,8-tetrahydrofolsäure der Formel II 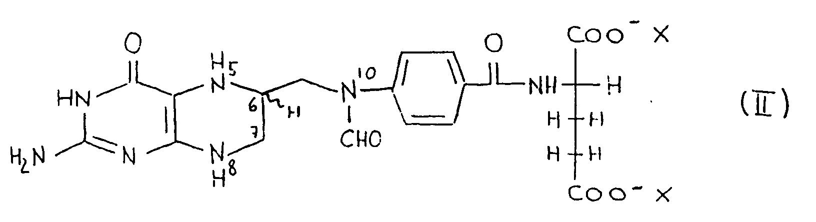 worin X ein Alkalimetallkation oder NH₄⁺ bedeutet,
vorzugsweise unter einer Inertgasatmosphäre, so lange mit wenigstens einer Säure S₁ vermischt, bis ein pH-Wert im Bereich von 5,5 bis 7,5 erhalten wird,
dann zum so erhaltenen Gemisch ein wasserlösliches Erdalkalimetallsalz hinzugibt,
anschliessend den entstandenen Festkörper A isoliert,
diesen Festkörper dann in Wasser suspendiert und unter Inertgasatmosphäre auf eine Temperatur von 70°C bis 100°C erhitzt und gleichzeitig den gemessenen pH-Wert mittels kontinuierlicher Hinzugabe einer wässrigen Lösung oder Suspension einer Base, ausgewählt aus der Gruppe, bestehend aus Erdalkalimetallhydroxiden, -carbonaten, und -hydrogencarbonaten, im Bereich von 5,0 bis 8,0 hält,
die erhaltene Lösung auf eine Temperatur von 30°C bis 50°C abkühlt und den gemessenen pH-Wert auf einen Wert von 6,5 bis 8,5 mittels Hinzugabe einer oben erwähnten Base erhöht,
diese Lösung etwa auf 3/4 des ursprünglichen Volumens bei einer Temperatur von mehr als 45°C einengt,
diese eingeengte, wenigstens 45°C warme Lösung langsam und kontrolliert während wenigstens 24 Stunden auf Raumtemperatur abkühlt, und
den erhaltenen Festkörper der Formel V abtrennt.

21. Verfahren nach Anspruch 20, dadurch gekennzeichnet, dass die Säure S₁ ausgewählt ist aus der Gruppe, bestehend aus niedrigen, wasserlöslichen Carbonsäuren, insbesondere Ameisensäure und Essigsäure, und Halogenwasserstoffsäuren, insbesondere HCl und HBr, wobei die genannten Säuren vorzugsweise als wässrige Lösungen verwendet werden.

22. Verfahren nach einem der Ansprüche 20 bis 21, dadurch gekennzeichnet, dass die Hinzugabe der Säure S₁ und des Erdalkalimetallsalzes bei einer Temperatur im Bereich von 0°C bis 60°C, insbesondere von 10°C bis 40°C, erfolgt.

23. Verfahren nach einem der Ansprüche 20 bis 22, dadurch gekennzeichnet, dass das Erdalkalimetallsalz ein entsprechendes Salz einer niedrigen, wasserlöslichen Carbonsäure, insbesondere ein Formiat oder ein Acetat, oder ein Halogenid, insbesondere ein Chlorid und Bromid, ist, wobei die entsprechenden Calcium- und Magnesiumsalze bevorzugt sind.

24. Verfahren nach einem der Ansprüche 20 bis 23, dadurch gekennzeichnet, dass das Erdalkalimetallsalz in einer Menge von 0,5 bis 1,0, insbesondere 0,7, Molequivalenten, bezogen auf die verwendete Menge an Verbindung der Formel II, verwendet wird.

25. Verfahren nach einem der Ansprüche 20 bis 24, dadurch gekennzeichnet, dass die Base Calcium- oder Magnesiumhydroxid ist.
